# EUROPEAN PATENT SPECIFICATION

(11) **EP 4 291 256 B1**
(45) Date of publication and mention of the grant of the patent: **18.02.2026**
(21) Application number: 22705146.3
(22) Date of filing: 09.02.2022
(51) Int. Cl.: A61L 24/00, A61L 24/04

(54) **TWO COMPONENT SEALING SYSTEMS INCLUDING SYNTHETIC MATRICES AND BIOSYNTHETIC ADHESIVES**
ZWEIKOMPONENTEN-DICHTUNGSSYSTEME MIT SYNTHETISCHEN MATRIZEN UND BIOSYNTHETISCHEN KLEBSTOFFEN
SYSTÈMES D'ÉTANCHÉITÉ À DEUX CONSTITUANTS COMPRENANT DES MATRICES SYNTHÉTIQUES ET DES ADHÉSIFS BIOSYNTHÉTIQUES

(30) Priority: 10.02.2021 US 202163148011 P
(43) Date of publication of application: 20.12.2023
(73) Proprietor: Ethicon, Inc, Raritan, NJ 08869 (US)
(72) Inventor: NATIV, Nir, Raritan, NJ 08869 (US); WEINDL, Thomas, Raritan, NJ 08869 (US); FITZ, Benjamin D., Raritan, NJ 08869 (US); LLANOS, Gerard, Raritan, NJ 08869 (US); ZHANG, Guanghui, Raritan, NJ 08869 (US); WANG, Yi-Lan, Raritan, NJ 08869 (US); DENG, Meng, Raritan, NJ 08869 (US)
(74) Representative: Carpmaels & Ransford LLP
(86) International application number: PCT/IB2022/051162
(87) International publication number: WO 2022/172169

(56) References cited:
- EP-A2- 2 179 753
- WO-A1-2021/009015
- US-A1- 2011 251 574
- US-A1- 2017 143 464
- US-A1- 2018 104 377

## Description

### BACKGROUND OF THE INVENTION

### Field of the Invention

The present patent application is generally related to systems and devices for use in surgical procedures, and is more specifically related to systems and devices for use in sealing tissue and organs to manage bleeding, fluid leaks, and air leaks.

### Description of the Related Art

A resection is a surgical procedure that involves cutting out tissue or a part of an organ. Resections, which are performed on a wide variety of organs including livers, lungs and gastrointestinal systems, present surgeons with many unique problems related to effectively managing post-operative bleeding, and fluid and air leaks.

During organ resection procedures, surgeons manage major bleeding at resected surfaces by using tourniquets, extensive suturing, etc. Some surgeons use adhesive fluids (e.g., synthetic adhesives, fibrin glue gels), which are limited since they can flow off the resected surface prior to completely curing or can peel off easy following curing since they lack adequate adherence to underlying tissue or proper elastic properties.

Liver Resection. A surgical procedure that involves removing all or part of a liver is commonly referred to as a hepatectomy. A partial hepatectomy is a preferred approach for removing a solid tumor from a liver. During resection of a liver, the tissue margins within the resected area are destroyed and the internal parenchyma and flow systems are exposed. The re-establishment of proper margins does not occur immediately, but requires an extended tissue healing process that can last from days to weeks. During the tissue healing process, the resected tissue can ooze blood and/or leak organ specific liquids (e.g., bile), which can cause post-operative complications. One study found that after liver resection procedures, the bile leakage rate was about 5%, which dramatically increased the likelihood of patient post-operative complications.

Lung Resection. Lung resection procedures typically require a significant amount of tissue manipulation and handling, which results in a high incidence of post-operative air leaks. Standardized techniques that are used to address air leaks involve suturing or stapling the lung tissue. These techniques are often ineffective, however, in creating an airtight seal due to the intrinsic friability of the lung parenchyma, particularly in emphysematous patients. In some instances, topical adhesives are applied either directly on the pleural abrasion or onto staple lines, however, these techniques are often inadequate for preventing air leaks.

GI Resection. Gastrointestinal (GI) procedures often involve excising large segments of a patient's intestinal anatomy for effectively treating disease. After removing GI tissue, surgeons are required to reconstruct the patient's digestive system. GI reconstructions are complicated due to the delicate structure of the intestines, limited blood supply to lower colon, limited surgeon access to complex anatomical structures, and the pathologies that typically affect the surrounding tissues. Even when surgeons exercise great care during GI reconstruction procedures, however, there are a certain percentage of patients who will have complications resulting from leaks at surgically created anastomotic sites. GI leaks can result in devastating outcomes, requiring additional surgeries and treatments that are often unsuccessful in managing the leaks.

In view of the above-noted complications, there have been many efforts directed to effectively managing intra and post-operative bleeding, and fluid and air leaks. For example, when resecting solid organs, surgeons typically manage major bleeding at resected surfaces by using tourniquets and extensive suturing. Minor bleeding and fluid leakage is often managed by using adhesive fluids (e.g., synthetic adhesives, fibrin glue) to cover the resected surfaces, however, these methodologies have achieved limited success because the adhesive fluids tend to flow off of the resected surfaces prior to curing and/or peel off after curing because the cured adhesive lacks adequate adherence to underlying tissue or proper elastic properties.

Covidien sells a VERISET^{®} hemostatic patch for sealing resected tissue surfaces. The VERISET^{®} hemostatic patch is composed of an oxidized regenerated cellulose (ORC) layer and a reactive polyethylene glycol (PEG) layer. The patch is applied to a tissue surface by applying pressure on the surface. Due to the fact that the ORC matrix is opaque, while applying pressure onto the resected surface, a surgeon cannot see through the VERISET^{®} hemostatic patch to assess the condition of the resected surface. The patch is also rigid with low flexibility and therefore may not achieve good tissue conformability or remain compliant with tissue movements.

US 2018/104377 A1 discusses a hemostatic composite sponge comprising oxidized cellulose and an essentially gelatin-free bioadhesive material stably associated with said sponge and present in an organized pattern on said sponge.

In view of the above-noted deficiencies, there is a continuing need for improved systems, devices and methods that enable surgeons to effectively seal resected surfaces of organ and tissue, and to prevent and/or successfully manage intra and post-operative bleeding, fluid leaks and/or air leaks from resected surfaces of tissue and organs.

To date, there have been many efforts directed to sealing the resected surfaces of organs to manage intra and post-operative bleeding, fluid leakage, and air leakage. Some of these efforts involve applying a mesh to a resected surface, followed by applying fibrin glue to seal the resected surface. Animal model experiments, however, indicate that fibrin glue does not have the tissue bonding strength to adhere the mesh to a resected surface of an organ.

### SUMMARY OF THE INVENTION

The present invention is directed to a system and process for sequentially depositing at least two different cross-linkable polyalkylene oxide-based components, preferably PEG-NHS and PEG-NH2, onto an absorbable matrix. In an aqueous environment, such as in the presence of blood: 1) the PEG-NHS and PEG-NH2 components tend to cross-link forming a gel-like mechanical barrier that may stop bleeding, and 2) the PEG-NHS cross-links with tissue proteins' NH2 groups, thereby leading to adhesion of the prototypes to the tissue. Notwithstanding this tissue adhesion function, Applicants discovered that with the selected matrix structure it was advantageous to deposit an excess of the PEG-NH2 component.

The inventors observed that the molar ratio of these two polyoxyalkylene oxide based reactive components is critical to the hemostatic efficacy of the patch. The potential reason for the molar ratio criticality is that two mechanisms, gel-formation and tissue adhesion, compete. Therefore, only at an optimal molar ratio of PEG-NHS and PEG-NH2 will the two mechanisms co-exist in a way that the prototype satisfies its hemostatic criteria.

In addition, the total PEGs density demonstrated a novel behavior, where an optimized range was identified for sufficient adherence to tissue. It was further discovered that the total quantity of the polyalkylene oxide reactive components had an upper limit as excess amounts formed a gel-like mechanical barrier largely impervious to the plasma/liquid phase which prevented integration of the resulting polymeric mass into the substrate.

The present patent application discloses preferred systems, devices and methods for overcoming the above-identified deficiencies and for their use in effectively sealing tissue to control bleeding, fluid leaks, and air leaks.

In one embodiment, the first component of the system is carrier matrix (e.g., mesh or non-woven) or substrate. In one embodiment, the matrix is a non-woven matrix. In one embodiment, the synthetic matrix is a biodegradable synthetic matrix.

In one embodiment, the matrix may be made from a synthetic substrate or patch such as an absorbable synthetic substrate made of a polyglactin 910 (PG910) material that is manufactured and sold under the trademark VICRYL^{®} polyglactin 910 material by Ethicon, Inc. of Somerville, New Jersey in a structured combination with a woven or non-woven layer of oxidized cellulose, preferably oxidized regenerated cellulose. In one embodiment, the polyglactin 910 material may be a copolymer material of about 90% glycolide and 10% L-lactide that is attached by needlepunching to a layer of woven oxidized cellulose material.

In one embodiment, the synthetic matrix may be a flexible or conformable non-woven matrix that is adapted to be placed onto the tissue surface of an organ and conform to the shape of the tissue surface and/or the organ. In one embodiment, the synthetic matrix is bioabsorbable.

In one embodiment, the wound dressing further includes at least two layers of reactive components that are co-reactive and sequentially deposited to minimize reaction. In one embodiment, the reactive components may include a biocompatible, reactive electrophile and a nucleophile. In one embodiment, the electrophile may include PEG-SG. In one embodiment, the nucleophile may be selected from any polymeric source of amine (NH2) groups (e.g., primary amine moieties), preferably polyethylene glycol amines (PEG-NH2), and combinations thereof.

The present invention is directed to hemostatic patches comprising a porous substrate having available acidic carboxylic groups and at least a pair of co-reactive polymer reagents comprising at least one nucleophilic polyalkylene oxide-based component and at least one electrophilic polyalkylene oxide-based component. The polymer reagent components are disposed on the porous substrate in a molar ratio of about 0.2 to about 0.9 : 1 of primary electrophilic (NHS) groups to nucleophilic (NH2) groups. At least one of said co-reactive polymer reagents can be a three-arm arm or four-arm polyethylene glycol-based nucleophile. At least one of said co-reactive polymer reagents can be a three-arm or four-arm polyethylene glycol-based electrophile. At least one electrophilic polyalkylene oxide based component can have an average molecular weight Mw of about 5kD to 30kD, preferably 10kD to 20kD. At least one nucleophilic polyalkylene oxide based component can have an average Mw of about 4kD to 20 kkD, preferably less than about 10kD, more preferably about 4kD-5kD. The electrophile component can be a polymeric material composed of polyethylene glycol succinimidyl glutarate ester (PEG-SG). The nucleophilic component can be a polymeric material selected from the group consisting of any polymeric source having available primary amine (NH2) groups.

A hemostatic patch can be characterized by having the porous substrate that is constructed primarily from materials of oxidized cellulose or oxidized regenerated cellulose. The porous substrate can be a bilayer construct having at least a first layer of an oxidized cellulose-containing material or oxidized regenerated cellulose-containing material with at least a second layer of a polyglactin-containing polymer. The porous substrate is preferably constructed from a blend of synthetic and cellulosic components, preferably in layers, biodegradable and flexible.

In one embodiment, the porous substrate comprises at least one layer of a non-woven mesh made of polyglactin 910 and a second attached layer of a non-woven mesh principally composed of oxidized cellulose or oxidized regenerated cellulosic material that provides localized acidic groups upon tissue application.

In one embodiment, the present invention is directed to methods for manufacturing a hemostatic patches as described herein where at least a pair of the co-reactive polymer reagents are applied to the substrate from a non-aqueous solvent spray or via an ultrasonic spray, as an example that can use air or gas. The non-aqueous solvent can be in whole or part an organic solvent, such as acetone. The at least a pair of the co-reactive polymer reagents can be applied sequentially with intervening drying step between application of a discreet layer of alternate co-reactive polymer reagent. The at least a pair of the co-reactive polymer are preferably applied in sequential and discreet layers of the first co-reactive polymer and the second co-reactive polymer. The layer of at least one polyethylene glycol-based electrophile can be applied as a first discreet layer in the sequence.

As will be set forth in more detail herein, it has been determined that the materials of construction of the non-woven blended matrix provides proper support and contributes localized acidic groups for the combination of reactive components to cure in a manner that will form a superior level of adhesion for forming a fluid and/or air-tight seal.

Additionally, it has been found that the sequentially deposition of the co-reactive adhesive components on an absorbable matrix under minimally aqueous environment produces suitable wound dressings. Further, Applicants discovered that controlling the molar ratio of the co-reactive adhesive components is critical to the hemostatic efficacy of the patch. Without being bound to the theory, it is believed that the preferred molar ratio of the co-reactive adhesive components balances two reaction mechanisms in which the polymeric electrophile, such as PEG-NHS, and polymeric nucleophile, such as PEG-NH2, tend to cross-link forming a gel-like mechanical barrier, and polymeric electrophile, such as PEG-NHS cross-links with tissue proteins' NH2 groups. In addition, the total density of co-reactive adhesive components demonstrated a novel behavior wherein the selected range provided sufficient adherence to tissue.

As will be disclosed in more detail herein, the order in which the first and second components are deposited on the substrate, as well as the particular composition of the biosynthetic adhesive, may affect the efficacy of the sealant, and thus can be modified and/or tailored for effective use on a particular application.

### DETAILED DESCRIPTION OF PREFERRED EMBODIMENTS

Polyethylene glycol succinimidyl glutarate (PEG-SG) has a well-established safety profile in medical devices and has been used in sealant products such as Duraseal and Coseal. The succinimidyl glutarate reacts with amine groups on proteins, e.g., collagen, under mildly alkaline conditions forming an amide bond. The cleavable ester linker designed into the PEG enables the polymer to be degradable in vivo. This form of PEG also provides the ability to cross-link across multiple collagen fibers due to its long spacer region allowing intermolecular cross-linking.

In one embodiment, the two reactive component sealant preferably includes a synthetic matrix that is adapted to be placed onto the tissue of a soft organ. In one embodiment, the synthetic matrix may be a mesh matrix or a non-woven matrix that is placed on the tissue surface together with the reactive components. In one embodiment, the non-woven matrix is flexible and/or conformable and is adapted for being placed onto a tissue surface, with the biosynthetic adhesive being applied to the synthetic matrix.

In one embodiment, the synthetic matrix preferably includes fibers and interstices that are disposed between the fibers.

In one embodiment, the biosynthetic or synthetic adhesive is applied to a biodegradable synthetic matrix (e.g., VICRYL^{®} non-woven PG910) in a manner that prevents substantial reaction of the co-reactive components. The biosynthetic adhesive components may be a solution of the PEG-NH2 and polyethylene glycol succinimidyl glutarate (PEG-SG) that is pre-mixed immediately prior to use in which one component has a protective leaving group that prevents reaction at the pH for delivery. Alternatively, the biosynthetic adhesive components can be provided on the matrix by sequential applications and drying of individual solutions of the co-reactive components. Still further, the biosynthetic adhesive components can be provided individually or as a blend onto the matrix in powder form.

Comparative Examples 1 and 2: Since PEG-NHS is an electrophile that would likely react with amine nucleophiles present in blood proteins and tissue, it was anticipated that coating PEG-NHS individually onto a textile substrate would promote adhesion of the substrate to a bleeding wound. However, it was found that applying only the electrophile, the PEG-NHS without the nucleophile PEG-NH2 was not effective at adhering a patch to a bleeding surface.

As an example, 16.0 mg/cm² of a 4-arm (10kDa) PEG-NHS was deposited onto a non-woven carboxylmethylcellulose (CMC) substrate, which was evaluated on a porcine spleen biopsy punch bleeding model. In this model the exemplary CMC patch was not effective in providing hemostatic efficacy.

Likewise, applying only the nucleophile, the PEG-NH2 without the electrophile PEG-NHS was not effective at adhering a patch to a bleeding surface. As an example, applying 6.5 mg/cm² of 4-arm (4kDa) PEG-NH2 onto a non-woven carboxymethylcellulose (CMC) substrate was not effective in providing hemostatic efficacy.

Apply 25.5 mg/cm² in total of PEGs (PEG-NHS, 4 arms at 10kDa [4ARM-SG-10K] and PEG-NH2 4 arms at 5kDa [4ARM-NH2-5000]) onto an 4 inch by 2 inch substrate composed of layer of woven oxidized regenerated cellulose and a non-woven absorbable polymer (PG910) (Bi-Layer Substrate). While the total PEGs loading density remained the same, the molar ratio of PEG-NHS to PEG-NH2 varied: 0.6, 1.2 and 1.8.

| Run # | PEG-NHS density mg/cm² | PEG-NH2 Density mg/cm² | Total PEG loading mg/cm² | PEG-NHS/ PEG-NH₂ Molar ratio |
|---|---|---|---|---|
| 3 | 13.9 | 11.6 | 25.5 | 0.6 |
| 1 | 18 | 7.5 | 25.5 | 1.2 |
| 2 | 20 | 5.5 | 25.5 | 1.8 |

The results of Applicants' studies indicate that the hemostatic efficacy decreases as the molar ratio of PEG-NHS to PEG-NH2 increases between 0.6 and 1.8. In addition, the results indicate that the PEGs molar ratio is a significant variable in the hemostatic performance of these prototypes in this model.

In addition, it was found that a molar ratio of PEG-NHS to PEG-NH₂ ratio was 0.25 (30.6 mg/cm² PEG-NH2, 19.2mg/cm² PEG-NHS) demonstrated hemostatic efficacy in a heparinized porcine bleeding model (Spleen, liver biopsy punch, and partial nephrectomy). (PEG Amine PEG-AM-4K; PEG-NHS- 4-arm N-hydroxysuccinimide glutarate ester terminated PEG, Mw~10,000g/mol, 4ARM-SG-10K)

Observations from 3 studies taken together enabled the inventors to discover that the molar ratio of the PEG does not follow a linear trend. When PEG-NHS to PEG-NH2 molar ratio approaches zero, the hemostatic efficacy of the prototype does not continue to increase compared to 0.25 and drops to zero hemostatic efficacy. When the PEG-NHS to PEG-NH2 molar ratio approaches infinity, the hemostatic efficacy drops to zero as well as the two PEGs are required to create a cohesive gel complex. The data supports an optimal PEG-NHS to PEG-NH2 molar ratio range of 0.25 to 1.8.

To demonstrate that samples within the suggested range of the PEG ratio are functional in a relevant bleeding acute animal model, the following study was performed. Test articles demonstrated hemostatic efficacy in a heparinized porcine bleeding model (Spleen biopsy punch, and spleen resection). PEG-NH2 (4-armed, and molecular weight of 4K) and PEG NHS (4-armed, n-hydroxysuccinimide glutarate ester terminated PEG, molecular weight of 10k, 4ARM-SG-10K). The substrate used was the Bi-Layer Substrate, and the acetone solution of each of components was sprayed ultrasonically onto the Bi-Layer Substrate: first the PEG-NH2 solution, and then the PEG-NHS solution. The coating levels and hemostasis models are listed below:

| | Hemostatic model | PEG-NHS density mg/ cm² | PEG-NH2 Density mg/ cm² | Total PEG loading mg/cm² | PEG-NHS/ PEG-NH2 Molar ratio |
|---|---|---|---|---|---|
| Test article 1 | Spleen biopsy punch (Mild to moderate bleeding) | 16 | 10 | 26 | 0.64 |
| Test article 2 | Spleen resection (Severe bleeding) | 16 | 14 | 30 | 0.46 |

PEG deposition density (mass of total PEGs deposited per substrate surface area) was evaluated, with a fixed molar ratio of 1.2:1.0 between succinimidyl groups PEG-SG and amine groups on PEG-Amine, for its effect on tissue peel force. Peel adhesion forces were evaluated for Bi-Layer prototypes with deposition densities ranging from 0 mg/cm² (uncoated) to 51.0 mg/cm² (200% of the standard S1 deposition density). Interestingly, peel force increased initially with increasing PEG deposition density, but plateaued at densities from 6.4 mg/cm² up to 25.5 mg/cm² (from 25% of standard S1 deposition density up to 100%). Beyond 25.5 mg/cm² deposition density, peel force begins to decline.

This trend suggests that the interfacial, chemical adhesion and subsequent gel formation of PEG-coated PEM to tissue is low, threshold-dependent on deposition density of PEG at the above fixed ratio, while above the threshold of 6.4 mg/cm², PEG layers have sufficient functionality to both adhere to tissue and form a strong, cross-linked gel with one another. However, above a PEGs density of 25.5 mg/cm² the ability to form a strong interconnected gel between the tissue surface and both PEGs may be hindered by the excessive spatial separation of the individual PEG layers.

| **PEG Deposition Density (mg/cm2)** | **Average Tissue Peel Force (N/m)** | **Standard Deviation (N/m)** |
|---|---|---|
| 0.0 | 4.47 | 0.48 |
| 1.3 | 81.00 | 17.22 |
| 3.8 | 146.89 | 16.43 |
| 6.4 | 186.23 | 27.08 |
| 12.8 | 193.27 | 31.62 |
| 25.5 | 180.70 | 32.33 |
| 51.0 | 91.30 | 6.27 |

### Example: Manufacturing Via Sequential Solution Spray (Ultrasonic)

In this process, a coating layer of an individual PEG is applied using an ultrasonic sprayer until target density of that PEG is reached. Dry air is used as a carrier gas for the atomized polymer solutions, though an inert gas can also be used such as nitrogen or argon. In one embodiment, the first coat of PEG-NH2 layers onto the matrix until reaching a target density, then drying the matrix under vacuum and subsequently coating layers of PEG-SG until reaching that respective target density. The fully-coated matrix is then dried under vacuum, and packaged.

The sealing system disclosed herein is independent of a patient's clotting system. Thus, surgeons can attain hemostasis and sealing independent of the patient's coagulation status, which could be impaired in patients being resected since most clotting proteins are produced in the liver. The sealing system may also be used for patients on antiplatelet and anticoagulant therapy, e.g., aspirin, heparin or warfarin.

In one embodiment, the electrophile may include PEG-SG. In one embodiment, the nucleophile may be selected from any polymeric source of NH₂ group, such as polyethylene glycol amines (PEG-NH2), and combinations of albumin and PEG-NH2.

## Claims

1. A hemostatic patch comprising a porous substrate having available acidic carboxylic groups and at least a pair of co-reactive polymer reagents comprising at least one nucleophilic polyalkylene oxide-based component and at least one electrophilic polyalkylene oxide-based component, both said components disposed on the porous substrate in a molar ratio of about 0.2 to about 0.9 : 1 of primary electrophilic groups to nucleophilic groups.

2. A hemostatic patch according to claim 1, wherein:
(i) at least one of said co-reactive polymer reagents is a three-arm arm polyethylene glycol-based nucleophile; or
(ii) at least one of said co-reactive polymer reagents is a three-arm arm polyethylene glycol-based electrophile; or
(iii) at least one of said co-reactive polymer reagents is a four-arm arm polyethylene glycol-based nucleophile; or
(iv) at least one of said co-reactive polymer reagents is a four-arm arm polyethylene glycol-based electrophile.

3. A method for manufacturing a hemostatic patch according to claim 1 wherein at least a pair of the co-reactive polymer reagents are applied to the substrate from a non-aqueous solvent spray via an ultrasonic air spray.

4. A method according to claim 3 wherein:
(i) the non-aqueous solvent comprises an organic solvent; or
(ii) the non-aqueous solvent comprises acetone.

5. A method according to claim 3 wherein at least a pair of the co-reactive polymer reagents are applied sequentially with intervening drying,
optionally wherein at least a pair of the co-reactive polymer are applied in sequential and discreet layers of the first co-reactive polymer and the second co-reactive polymer,
further optionally wherein the layer of at least one polyethylene glycol-based electrophile is applied as a first layer in sequence.

6. A hemostatic patch according to claim 1 wherein the porous substrate comprises oxidized cellulose or oxidized regenerated cellulose.

7. A hemostatic patch according to claim 1 wherein the porous substrate is a bilayer construct that comprises at least a first layer of an oxidized cellulose-containing material or oxidized regenerated cellulose-containing material with at least a second layer of a polyglactin-containing polymer.

8. A hemostatic patch according to claim 1 wherein said at least one electrophilic polyalkylene oxide based component has an average molecular weight Mw of about 10kD.

9. A hemostatic patch according to claim 1 wherein said at least one nucleophilic polyalkylene oxide based component has an average Mw of about 4kD to 5kD.

10. The hemostatic patch according to claim 1, wherein said porous substrate is a synthetic, biodegradable and flexible matrix.

11. The hemostatic patch according to claim 10, wherein said porous substrate comprises at least one layer of a non-woven mesh made of polyglactin 910 and a second attached layer of a non-woven mesh consisting essentially of oxidized cellulose or oxidized regenerated cellulosic material that provides localized acidic groups upon tissue application.

12. The hemostatic patch according to claim 1, wherein the electrophile component comprises a polyethylene glycol succinimidyl glutarate ester (PEG-SG).

13. The hemostatic patch according to claim 1, wherein the nucleophilic component is selected from the group consisting of any polymeric source of primary amine (NH₂) groups.

## Patentansprüche

1. Hämostatischer Patch, umfassend ein poröses Substrat mit verfügbaren sauren Carboxylgruppen und mindestens ein Paar co-reaktiver Polymerreagenzien, umfassend mindestens eine nucleophile Polyalkylenoxid-basierte Komponente und mindestens eine elektrophile Polyalkylenoxid-basierte Komponente, wobei beide Komponenten auf dem porösen Substrat in einem Molverhältnis von etwa 0,2 bis etwa 0,9:1 von primären elektrophilen Gruppen zu nucleophilen Gruppen angeordnet sind.

2. Hämostatischer Patch nach Anspruch 1, wobei:
(i) mindestens eines der co-reaktiven Polymerreagenzien ein dreiarmiges Polyethylenglycol-basiertes Nucleophil ist; oder
(ii) mindestens eines der co-reaktiven Polymerreagenzien ein dreiarmiges Polyethylenglycol-basiertes Elektrophil ist; oder
(iii) mindestens eines der co-reaktiven Polymerreagenzien ein vierarmiges Polyethylenglycol-basiertes Nucleophil ist; oder
(iv) mindestens eines der co-reaktiven Polymerreagenzien ein vierarmiges Polyethylenglycol-basiertes Elektrophil ist.

3. Verfahren zum Herstellen eines hämostatischen Patch nach Anspruch 1, wobei mindestens ein Paar der co-reaktiven Polymerreagenzien auf das Substrat aus einem nicht-wässrigen Lösungsmittelspray mittels eines Ultraschall-Luftsprays aufgebracht wird.

4. Verfahren nach Anspruch 3, wobei:
(i) das nicht-wässrige Lösungsmittel ein organisches Lösungsmittel umfasst; oder
(ii) das nicht-wässrige Lösungsmittel Aceton umfasst.

5. Verfahren nach Anspruch 3, wobei mindestens ein Paar der co-reaktiven Polymerreagenzien sequenziell mit zwischenliegender Trocknung aufgebracht wird,
wobei gegebenenfalls mindestens ein Paar des co-reaktiven Polymers in aufeinanderfolgenden und diskreten Schichten des ersten co-reaktiven Polymers und des zweiten co-reaktiven Polymers aufgebracht wird,
wobei ferner gegebenenfalls die Schicht aus mindestens einem Polyethylenglycol-basierten Elektrophil als erste Schicht in der Reihenfolge aufgebracht wird.

6. Hämostatischer Patch nach Anspruch 1, wobei das poröse Substrat oxidierte Cellulose oder oxidierte regenerierte Cellulose umfasst.

7. Hämostatischer Patch nach Anspruch 1, wobei das poröse Substrat ein Zweischichtkonstrukt ist, das mindestens eine erste Schicht aus einem oxidierten cellulosehaltigen Material oder oxidierten regenerierten cellulosehaltigen Material mit mindestens einer zweiten Schicht aus einem polyglactinhaltigen Polymer umfasst.

8. Hämostatischer Patch nach Anspruch 1, wobei die mindestens eine elektrophile Polyalkylenoxid-basierte Komponente ein durchschnittliches Molekulargewicht Mw von etwa 10 kD aufweist.

9. Hämostatischer Patch nach Anspruch 1, wobei die mindestens eine nucleophile Polyalkylenoxid-basierte Komponente ein durchschnittliches Mw von etwa 4 kD bis 5 kD aufweist.

10. Hämostatischer Patch nach Anspruch 1, wobei das poröse Substrat eine synthetische, biologisch abbaubare und flexible Matrix ist.

11. Hämostatischer Patch nach Anspruch 10, wobei das poröse Substrat mindestens eine Schicht eines Vlies-Netzes aus Polyglactin 910 und eine zweite befestigte Schicht eines Vlies-Netzes umfasst, das im Wesentlichen aus oxidierter Cellulose oder oxidiertem regeneriertem cellulosischem Material besteht, das bei Gewebeauftrag lokalisierte saure Gruppen bereitstellt.

12. Hämostatischer Patch nach Anspruch 1, wobei die elektrophile Komponente einen Polyethylenglycol-Succinimidylglutarat-Ester (PEG-SG) umfasst.

13. Hämostatischer Patch nach Anspruch 1, wobei die nucleophile Komponente ausgewählt ist aus der Gruppe bestehend aus einer beliebigen polymeren Quelle primärer Amin-(NH₂)-Gruppen.

## Revendications

1. Patch hémostatique comprenant un substrat poreux ayant des groupes carboxyliques acides disponibles et au moins une paire de réactifs polymères coréactifs comprenant au moins un composant nucléophile à base d'oxyde de polyalkylène et au moins un composant électrophile à base d'oxyde de polyalkylène, lesdits deux composants étant disposés sur le substrat poreux dans un rapport molaire d'environ 0,2 à environ 0,9 : 1 des groupes électrophiles primaires aux groupes nucléophiles.

2. Patch hémostatique selon la revendication 1, dans lequel :
(i) au moins l'un desdits réactifs polymères co-réactifs est un nucléophile à base de polyéthylène glycol à trois bras ; ou
(ii) au moins l'un desdits réactifs polymères coréactifs est un électrophile à base de polyéthylène glycol à trois bras ; ou
(iii) au moins l'un desdits réactifs polymères est un nucléophile à base de polyéthylène glycol à quatre bras ; ou
(iv) au moins l'un desdits réactifs polymères coréactifs est un électrophile à base de polyéthylène glycol à quatre bras.

3. Procédé de fabrication d'un patch hémostatique selon la revendication 1, dans lequel au moins une paire de réactifs polymères coréactifs est appliquée sur le substrat à partir d'une pulvérisation de solvant non aqueux par l'intermédiaire d'une pulvérisation d'air ultrasonique.

4. Procédé selon la revendication 3, dans lequel :
(i) le solvant non aqueux comprend un solvant organique ; ou
(ii) le solvant non aqueux comprend de l'acétone.

5. Procédé selon la revendication 3, dans lequel au moins une paire de réactifs polymères coréactifs est appliquée séquentiellement avec un séchage intermédiaire,
éventuellement, dans lequel au moins une paire du polymère coréactif est appliquée en couches séquentielles et distinctes du premier polymère coréactif et du second polymère coréactif,
éventuellement, en outre dans lequel la couche d'au moins un électrophile à base de polyéthylène glycol est appliquée en guise de première couche en séquence.

6. Patch hémostatique selon la revendication 1, dans lequel le substrat poreux comprend de la cellulose oxydée ou de la cellulose régénérée oxydée.

7. Patch hémostatique selon la revendication 1, dans lequel le substrat poreux est une construction bicouche qui comprend au moins une première couche d'un matériau contenant de la cellulose oxydée ou un matériau contenant de la cellulose régénérée oxydée avec au moins une seconde couche d'un polymère contenant de la polyglactine.

8. Patch hémostatique selon la revendication 1, dans lequel ledit au moins un composant électrophile à base d'oxyde de polyalkylène a une masse moléculaire moyenne Mw d'environ 10 kD.

9. Patch hémostatique selon la revendication 1, dans lequel ledit au moins un composant à base d'oxyde de polyalkylène nucléophile a une Mw moyenne d'environ 4 kD à 5 kD.

10. Patch hémostatique selon la revendication 1, dans lequel ledit substrat poreux est une matrice synthétique, biodégradable et flexible.

11. Patch hémostatique selon la revendication 10, dans lequel ledit substrat poreux comprend au moins une couche d'une maille non tissée faite de polyglactine 910 et une seconde couche fixée d'une maille non tissée constituée essentiellement de cellulose oxydée ou de matériau cellulosique régénéré oxydé qui fournit des groupes acides localisés lors de l'application tissulaire.

12. Patch hémostatique selon la revendication 1, dans lequel le composant électrophile comprend un succinimidylique glutarate ester de polyéthylène glycol (PEG-SG).

13. Patch hémostatique selon la revendication 1, dans lequel le composant nucléophile est choisi dans le groupe constitué par l'une quelconque source polymère de groupes d'amine primaire (NH₂).
